# EUROPEAN PATENT SPECIFICATION

(11) **EP 3 284 595 B1**
(45) Date of publication and mention of the grant of the patent: **21.02.2024**
(21) Application number: 16780037.4
(22) Date of filing: 12.04.2016
(51) Int. Cl.: B32B 27/12, B32B 25/10, C09J 7/00, C09J 123/02, A61F 13/15

(54) **EXPANDING/CONTRACTING LAMINATE AND PRODUCT INCLUDING SAME**
EXPANDIERENDES/KONTRAHIERENDES LAMINAT UND PRODUKT DAMIT
STRATIFIÉ À EXPANSION/CONTRACTION ET PRODUIT COMPRENANT CELUI-CI

(30) Priority: 15.04.2015 JP 2015082963; 23.03.2016 JP 2016058093
(43) Date of publication of application: 21.02.2018
(73) Proprietor: Nitto Denko Corporation, Ibaraki-shi, Osaka 567-8680 (JP)
(72) Inventor: UCHIDA, Shou, Ibaraki-shi Osaka 567-8680 (JP); TAKEDA, Kohei, Ibaraki-shi Osaka 567-8680 (JP); NAKAGAWA, Muneshige, Ibaraki-shi Osaka 567-8680 (JP); IKISHIMA, Shinsuke, Ibaraki-shi Osaka 567-8680 (JP)
(74) Representative: Müller-Boré & Partner Patentanwälte PartG mbB
(86) International application number: PCT/JP2016/061797
(87) International publication number: WO 2016/167246

(56) References cited:
- WO-A1-2014/025849
- WO-A1-2015/008595
- JP-A- 2008 213 284
- JP-A- 2014 180 461
- US-A1- 2004 121 687
- US-A1- 2008 003 911
- US-A1- 2010 215 923

## Description

The present invention relates to a stretchable laminate and an article including the stretchable laminate.

Various stretchable laminates have been proposed as members for articles such as sanitary articles, for example, a diaper and a mask (see, for example, Patent Literatures 1 and 2) . For example, WO 2015/0085951 discloses a stretchable laminate and an article containing the same. Furthermore, US 2010/215923 A1 discloses elastic film laminates with tapered point bonds. Still further, US 2004/121687 A1 discloses an extendible laminate having improved stretch properties and a method for making the same.

A stretchable laminate formed of two or more layers including an elastomer layer has been proposed as such member. Typically, a stretchable laminate having a non-woven fabric layer on at least one side of an elastomer layer has been proposed. In such stretchable laminate, the elastomer layer and the non-woven fabric layer are generally bonded to each other with an adhesive or a pressure-sensitive adhesive.

However, such related-art stretchable laminate formed of two or more layers including an elastomer layer involves a problem in that delamination occurs between the elastomer layer and a layer adjacent thereto. In addition, an adhesive or a pressure-sensitive adhesive is used in the laminate, and hence the laminate involves a problem in that a unique odor derived from the adhesive or the pressure-sensitive adhesive occurs and a problem in that its cost increases. Further, even when the elastomer layer and the layer adjacent thereto each have air permeability, the bonding of the elastomer layer and the adjacent layer with the adhesive or the pressure-sensitive adhesive causes a problem in that the air permeability is inhibited.

[PTL 1] JP 2012-187857 A
[PTL 2] JP 3830818 B2
[PTL 3] WO 2015/0085951
[PTL 4] US 2010/215923 A1
[PTL 5] US 2004/121687 A1

The present invention has been made to solve the conventional problems, and an object of the present invention is to provide a stretchable laminate formed of more than two layers including an elastomer layer, the stretchable laminate having the following features: delamination hardly occurs between the elastomer layer and a layer adjacent thereto; the occurrence of a unique odor derived from an adhesive or a pressure-sensitive adhesive is suppressed; the inhibition of air permeability due to the bonding of the elastomer layer and the layer adj acent thereto can be prevented; and the laminate can be produced at lower cost than ever before. Another object of the present invention is to provide an article including such stretchable laminate.

The present invention is defined in the claims. According to one embodiment of the present invention, there is provided a stretchable laminate, including more than two layers including an elastomer layer 10, a non-woven fabric layer 20a arranged on one side of the elastomer layer, and a non-woven fabric layer 20b arranged on the elastomer layer on an opposite side to the non-woven fabric layer, in which the elastomer layer 10, the non-woven fabric layer 20a, and the non-woven fabric layer 20b are directly laminated,
wherein the stretchable laminate has a region C having through-holes,
wherein the elastomer layer 10, the non-woven fabric layer 20a, and the non-woven fabric layer 20b are directly welded and bonded to each other,
wherein the welding bonding comprises ultrasonic welding bonding, and
wherein the through-holes in the region C are formed by feeding an ultrasonic wave from a horn so that vibration energy is more intensively applied to sites where the through-holes are to be formed at the time of bonding in the region C.

In a preferred embodiment, the elastomer layer has a three-layer structure.

In a preferred embodiment, the three-layer structure has, as an intermediate layer, a layer in which two or more kinds of elastomers are blended, and has, as both surface layers, layers each containing one of elastomers of the same kinds as the elastomers in the intermediate layer.

In a preferred embodiment, the elastomer layer has a thickness of from 20 µm to 200 µm.

In a preferred embodiment, the elastomer layer has a thickness of from 30 µm to 100 µm.

In a preferred embodiment, the elastomer layer contains an olefin-based elastomer.

In a preferred embodiment, the olefin-based elastomer includes an α-olefin-based elastomer.

In a preferred embodiment, the α-olefin-based elastomer includes at least one kind selected from an ethylene-based elastomer, a propylene-based elastomer, and a 1-butene-based elastomer.

In a preferred embodiment, the non-woven fabric layer 20a and the non-woven fabric layer 20b contain fibers of polyolefin.

In a preferred embodiment, the polyolefin includes polypropylene.

In a preferred embodiment, the non-woven fabric layer 20a and the non-woven fabric layer 20b are formed of a non-woven fabric having a basis weight of 150 gsm or less.

In a preferred embodiment, the non-woven fabric 20a and the non-woven fabric layer 20b have a basis weight of 50 gsm or less.

In a preferred embodiment, the non-woven fabric (20a) and the non-woven fabric layer (20b) have a basis weight of from 10 gsm to 30 gsm.

In a preferred embodiment, the stretchable laminate according to the embodiment of the present invention has a region A free of a through-hole in one end portion thereof and a region B free of a through-hole in another end portion thereof, and has the region C having the through-holes between the region A and the region B.

According to another embodiment of the present invention, there is provided an article, including the stretchable laminate according to the embodiment of the present invention.

According to the present invention, the stretchable laminate formed of more than two layers including an elastomer layer, the stretchable laminate having the following features can be provided: delamination hardly occurs between the elastomer layer and a layer adjacent thereto; the occurrence of a unique odor derived from an adhesive or a pressure-sensitive adhesive is suppressed; the inhibition of air permeability due to the bonding of the elastomer layer and the layer adjacent thereto can be prevented; and the laminate can be produced at lower cost than ever before . The article including such stretchable laminate can also be provided.

FIG. 1 is a schematic sectional view of a stretchable laminate .
FIG. 2 is a schematic sectional view of another stretchable laminate according to an embodiment of the present invention.
FIG. 3 is a schematic sectional view of still another stretchable laminate according to an embodiment of the present invention.
FIG. 4 is a schematic plan view of the still other stretchable laminate according to an embodiment of the present invention of FIG. 3.
FIG. 5 is a schematic sectional view of still another stretchable laminate according to a preferred embodiment of the present invention.
FIG. 6 is a schematic plan view of the still other stretchable laminate according to the preferred embodiment of the present invention of FIG. 5.

### <<<<Stretchable Laminate>>>>

A stretchable laminate of the present invention is a stretchable laminate formed of more than two layers including an elastomer layer. The stretchable laminate of the present invention may include any appropriate other layer except the elastomer layer to the extent that the effects of the present invention are not impaired as long as the stretchable laminate is formed of more than two layers including the elastomer layer. The number of such any appropriate other layers may be two or more.

In the stretchable laminate of the present invention, the elastomer layer 10, the non-woven fabric layers 20a, and the non-woven fabric layer 20b are directly laminated. That is, the foregoing means that in the stretchable laminate of the present invention, the elastomer layer 10, the non-woven fabric layers 20a, and the non-woven fabric layer 20b are directly laminated without intermediation of any other layer, such as an adhesive layer or a pressure-sensitive adhesive layer, between the two layers. With such construction, the stretchable laminate of the present invention has the following features: delamination hardly occurs between the elastomer layer and a layer adjacent thereto; the occurrence of a unique odor derived from an adhesive or a pressure-sensitive adhesive is suppressed; the inhibition of air permeability due to the bonding of the elastomer layer and the layer adjacent thereto can be prevented; and the laminate can be produced at lower cost than ever before.

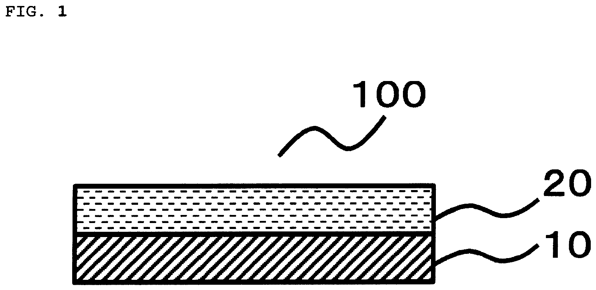

FIG. 1 is a schematic sectional view of a stretchable laminate. A stretchable laminate 100 illustrated in FIG. 1 includes an elastomer layer 10 and a non-woven fabric layer 20 arranged on only one side of the elastomer layer 10. In the stretchable laminate 100 illustrated in FIG. 1, the elastomer layer 10 and the non-woven fabric layer 20 are directly laminated.

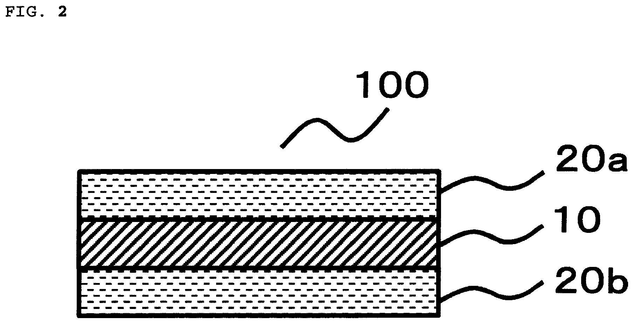

FIG. 2 is a schematic sectional view of another stretchable laminate. A stretchable laminate 100 illustrated in FIG. 2 has an elastomer layer 10, a non-woven fabric layer 20a arranged on one side of the elastomer layer 10, and a non-woven fabric layer 20b arranged on the elastomer layer 10 on an opposite side to the non-woven fabric layer 20a. In the stretchable laminate 100 illustrated in FIG. 2, the elastomer layer 10 and the non-woven fabric layer 20a are directly laminated. In the stretchable laminate 100 illustrated in FIG. 2, the elastomer layer 10 and the non-woven fabric layer 20b are directly laminated.

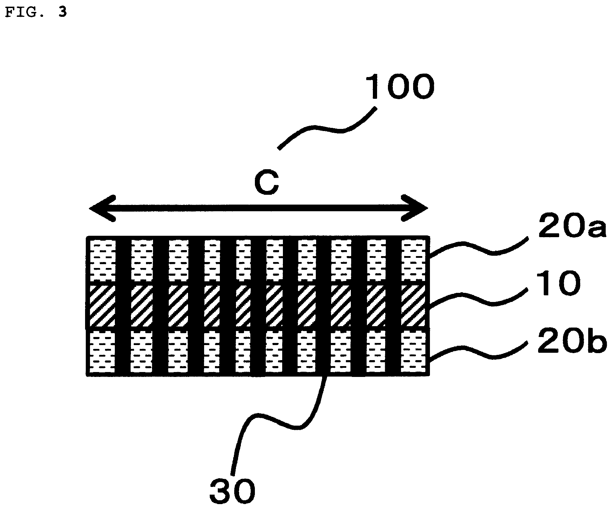

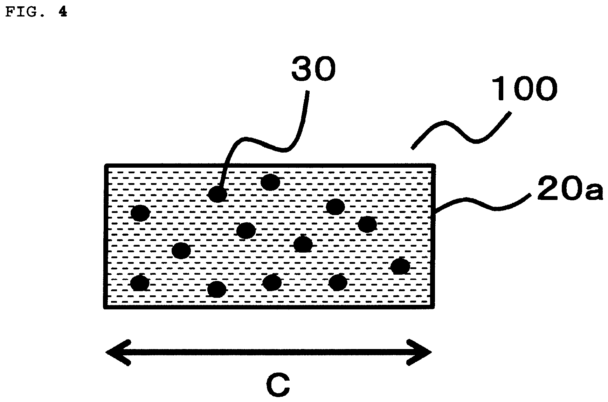

FIG. 3 is a schematic sectional view of a stretchable laminate according to the present invention. A stretchable laminate 100 illustratedin FIG. 3 includes an elastomer layer 10, a non-woven fabric layer 20a arranged on one side of the elastomer layer 10, and a non-woven fabric layer 20b arranged on the elastomer layer 10 on an opposite side to the non-woven fabric layer 20a, and the laminate has a region C having through-holes 30 . A schematic plan view of the stretchable laminate 100 illustrated in FIG. 3 is FIG. 4. The stretchable laminate 100 illustrated in FIG. 4 has a region C having through-holes 30.

Such stretchable laminate as illustrated in each of FIG. 3 and FIG. 4 has the region C having the through-holes. With such construction, the stretchable laminate of the present invention has the through-holes in the region C, and hence can express more excellent air permeability in the region C.

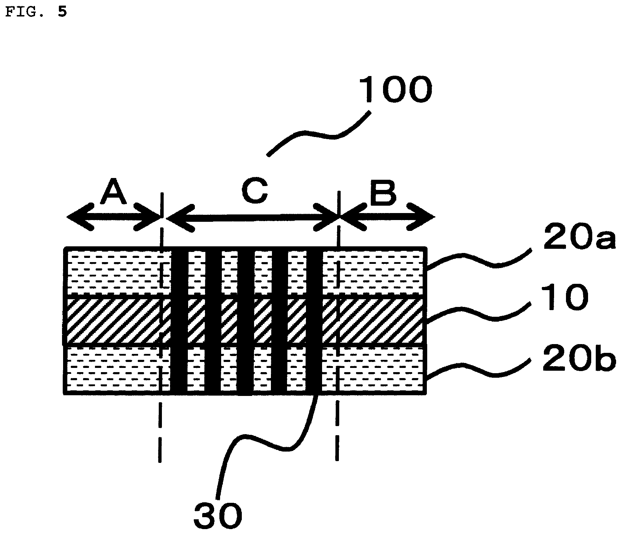

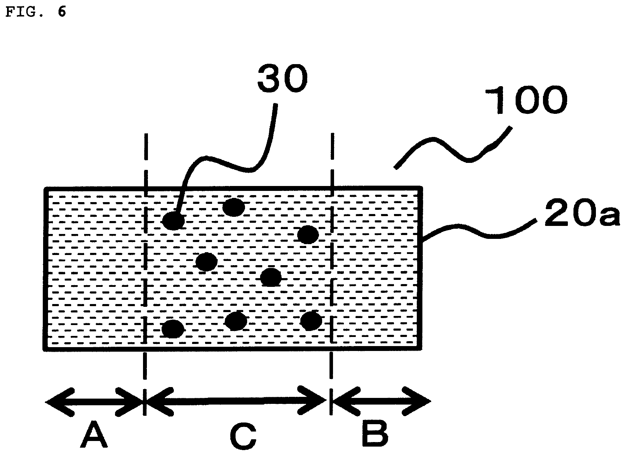

FIG. 5 is a schematic sectional view of still another stretchable laminate according to a preferred embodiment of the present invention. A stretchable laminate 100 illustrated in FIG. 5 includes an elastomer layer 10, a non-woven fabric layer 20a arranged on one side of the elastomer layer 10, and a non-woven fabric layer 20b arranged on the elastomer layer 10 on an opposite side to the non-woven fabric layer 20a, and the laminate has a region A free of a through-hole in one end portion thereof and a region B free of a through-hole in the other end portion thereof, and has a region C having through-holes 30 between the region A and the region B. A schematic plan view of the stretchable laminate 100 illustrated in FIG. 5 is FIG. 6. The stretchable laminate 100 illustrated in FIG. 6 has the region A free of a through-hole in one end portion thereof and the region B free of a through-hole in the other end portion thereof, and has the region C having the through-holes 30 between the region A and the region B.

Such stretchable laminate as illustrated in each of FIG. 5 and FIG. 6 has the region A free of a through-hole in one end portion thereof and the region B free of a through-hole in the other end portion thereof, and has the region C having the through-holes between the region A and the region B. With such construction, the stretchable laminate of the present invention has the through-holes in the region C, and hence can express more excellent air permeability in the region C. In addition, the stretchable laminate of the present invention has the region A free of a through-hole and the region B free of a through-hole in its end portions, and hence the end portions can be further strengthened.

The thickness of the stretchable laminate of the present invention varies depending on the thickness of the elastomer layer or the thickness of any other layer, such as the non-woven fabric layer, and is preferably from 1.0 mm to 0.1 mm, more preferably from 0.8 mm to 0.15 mm, still more preferably from 0.6 mm to 0.15 mm, particularly preferably from 0.5 mm to 0.2 mm, most preferably from 0.45 mm to 0.2 mm. When the thickness of the stretchable laminate of the present invention falls within such range, the laminate can be easily used as a material used in articles such as sanitary articles, for example, a diaper and a mask.

In the stretchable laminate of the present invention, the elastomer layer 10, the non-woven fabric layer 20a, and the non-woven fabric layer 20b directly fused and bonded to each other, and the welding bonding include ultrasonic welding bonding.

Since the elastomer layer and the layer adjacent thereto are directly fused and bonded to each other by the ultrasonic welding bonding, the stretchable laminate of the present invention has the following features: the delamination more hardly occurs between the elastomer layer and the layer adjacent thereto; the occurrence of the unique odor derived from the adhesive or the pressure-sensitive adhesive is further suppressed; the inhibition of the air permeability due to the bonding of the elastomer layer and the layer adjacent thereto can be further prevented; and the laminate can be produced at even lower cost than ever before.

In the stretchable laminate of the present invention, the elastomer layer and the layer adjacent thereto preferably contain the same kind of material (e.g. , a polyolefin-based elastomer layer and a polyolefin-based adj acent layer) , and more preferably contain, as their main components, the same kind of material. When the same kind of material is used, the delamination more hardly occurs between the elastomer layer and the layer adjacent thereto.

Any appropriate ultrasonic welding bonding may be adopted as the ultrasonic welding bonding to the extent that the effects of the present invention are not impaired.

In the ultrasonic welding bonding, members to be bonded (e. g. , two or more layer members including the elastomer layer) are arranged between a part generally referred to as "horn", the part being configured to feed vibration energy with an ultrasonic wave, and a roll-shaped part generally referred to as "anvil". In many cases, the horn is arranged vertically above the members to be bonded and the anvil. The horn typically vibrates at from 20,000 Hz to 40,000 Hz to transfer energy typically in the form of frictional heat to the members to be bonded under pressure. Part of at least one of the members to be bonded is softened or melted by the frictional heat and the pressure, and hence the materials are bonded to each other.

One preferred kind of ultrasonic welding bonding is generally known as "continuous ultrasonic welding bonding." The continuous ultrasonic welding bonding is typically used for sealing members to be bonded that can be supplied into a bonding apparatus in a substantially continuous manner. In the continuous ultrasonic welding bonding, the horn is typically fixed and the members to be bonded move directly below the horn. In one kind of continuous ultrasonic welding bonding, the fixed horn and a rotating anvil surface are used. During the continuous ultrasonic welding bonding, the members to be bonded are pulled between the horn and the rotating anvil. The horn typically extends in its lengthwise direction toward the members to be bonded, and its vibration moves along the horn in its axial direction to the materials.

In another preferred kind of ultrasonic welding bonding, the horn is a rotation type, has a cylindrical shape, and rotates about its lengthwise direction axis. Input vibration is present in the axial direction of the horn and output vibration is present in the radial direction of the horn. The horn is arranged so as to be close to the anvil, and the anvil can also typically rotate so that the members to be bonded may pass a space between cylindrical surfaces at a line velocity substantially equal to the tangential velocity of the cylindrical surfaces.

The ultrasonic welding bonding is disclosed in, for example, JP 2008-526552 A, JP 2010-195044 A, JP 2013-231249 A, JP 2015-16294 A, and US 5976316 A.

### <<Elastomer Layer>>

Any appropriate elastomer layer may be adopted as the elastomer layer to the extent that the effects of the present invention are not impaired. Examples of an elastomer resin serving as a main component of such elastomer layer include an olefin-based elastomer, a styrene-based elastomer, a vinyl chloride-based elastomer, a urethane-based elastomer, an ester-based elastomer, and an amide-based elastomer.

The content of the elastomer resin serving as the main component in the elastomer layer is preferably from 50 wt% to 100 wt%, more preferably from 70 wt% to 100 wt%, still more preferably from 90 wt% to 100 wt%, particularly preferably from 95 wt% to 100 wt%, most preferably from 98 wt% to 100 wt%. When the content of the elastomer resin serving as the main component in the elastomer layer falls within the range, the elastomer layer can express a sufficient elastomer characteristic.

The number of the elastomer layers may be one, or may be two or more. When the elastomer layer has a three-layer structure, the three-layer structure is preferably, for example, such a three-layer structure that a layer in which two or more kinds of elastomers are blended is used as an intermediate layer, and layers each containing one of elastomers of the same kinds as the elastomers in the intermediate layer are used as both surface layers.

In the present invention, the elastomer resin serving as the main component in the elastomer layer is preferably an olefin-based elastomer. When the olefin-based elastomer is adopted as the elastomer resin, heat stability is improved as compared to any other elastomer resin (e.g., a styrene-based elastomer), and hence, for example, heat decomposition at the time of the formation of the resin into a film in the production of the stretchable laminate of the present invention can be suppressed. In addition, when the olefin-based elastomer is adopted as the elastomer resin, storage stability is improved as compared to any other elastomer resin (e.g. , a styrene-based elastomer), and hence the fluctuation of values for physical properties during the storage of the stretchable laminate of the present invention can be suppressed.

In the present invention, when the olefin-based elastomer is adopted as the elastomer resin, steps in the production of the elastomer layer can be simplified, and hence processing cost can be suppressed. This is because of, for example, the following reason. When any other elastomer resin (e.g., a styrene-based elastomer) is adopted as the elastomer resin, several kinds of styrene-based elastomers need to be blended for controlling values for physical properties . To this end, a master batch needs to be produced. When the olefin-based elastomer is adopted as the elastomer resin, extrusion molding can be performed by using fewer kinds of resins in the production of the elastomer layer, and hence the need for the production of the master batch can be eliminated.

In the present invention, when the olefin-based elastomer is adopted as the elastomer resin, the olefin-based elastomer may be only one kind of elastomer or a blend of two or more kinds of elastomers .

Examples of the olefin-based elastomer include an olefin block copolymer, an olefin random copolymer, an ethylene copolymer, a propylene copolymer, an ethylene olefin block copolymer, a propylene olefin block copolymer, an ethylene olefin random copolymer, a propylene olefin random copolymer, an ethylene propylene random copolymer, an ethylene (1-butene) random copolymer, an ethylene (1-pentene) olefin block copolymer, an ethylene (1-hexene) random copolymer, an ethylene (1-heptene) olefin block copolymer, an ethylene (1-octene) olefin block copolymer, an ethylene (1-nonene) olefin block copolymer, an ethylene (1-decene) olefin block copolymer, a propylene ethylene olefin block copolymer, an ethylene (α-olefin) copolymer, an ethylene (α-olefin) random copolymer, an ethylene (α-olefin) block copolymer, amorphous polypropylene, combinations of the above-mentioned polymers and polyethylene (LLDPE, LDPE, HDPE, or the like), combinations of the above-mentioned polymers and polypropylene, and combinations thereof.

The olefin-based elastomer that may be adopted as the elastomer resin in the present invention has a density of preferably from 0.890 g/cm³ to 0.830 g/cm³, more preferably from 0.888 g/cm³ to 0.835 g/cm³, still more preferably from 0.886 g/cm³ to 0.835 g/cm³, particularly preferably from 0.885 g/cm³ to 0.840 g/cm³, most preferably from 0.885 g/cm³ to 0.845 g/cm³. When the olefin-based elastomer whose density falls within the range is adopted, a stretchable laminate having more excellent fittability can be provided. In addition, the heat stability is further improved as compared to any other elastomer resin (e.g., a styrene-based elastomer), and hence, for example, the heat decomposition at the time of the formation of the resin into a film in the production of the stretchable laminate of the present invention can be further suppressed. In addition, the storage stability is further improved as compared to any other elastomer resin (e.g., a styrene-based elastomer), and hence the fluctuation of values for physical properties during the storage of the stretchable laminate of the present invention can be further suppressed. Further, the steps in the production of the elastomer layer can be further simplified, and hence the processing cost can be further suppressed.

The olefin-based elastomer that may be adopted as the elastomer resin in the present invention has a MFR at 230°C and 2.16 kgf of preferably from 1.0 g/10 min to 25.0 g/10 min, more preferably from 2.0 g/10 min to 23.0 g/10 min, still more preferably from 2.0 g/10 min to 21.0 g/10 min, particularly preferably from 2.0 g/10 min to 20.0 g/10 min, most preferably from 2.0 g/10 min to 19.0 g/10 min. When the olefin-based elastomer whose MFR falls within the range is adopted, a stretchable laminate having more excellent fittability can be provided. In addition, the heat stability is further improved as compared to any other elastomer resin (e.g., a styrene-based elastomer), and hence, for example, the heat decomposition at the time of the formation of the resin into a film in the production of the stretchable laminate of the present invention can be further suppressed. In addition, the storage stability is further improved as compared to any other elastomer resin (e.g., a styrene-based elastomer), and hence the fluctuation of values for physical properties during the storage of the stretchable laminate of the present invention can be further suppressed. Further, the steps in the production of the elastomer layer can be further simplified, and hence the processing cost can be further suppressed.

The olefin-based elastomer that may be adopted as the elastomer resin in the present invention is specifically preferably an α-olefin-based elastomer. Of such α-olefin-based elastomers, at least one kind selected from an ethylene-based elastomer, a propylene-based elastomer, and a 1-butene-based elastomer is more preferred. When such α-olefin-based elastomer is adopted as the olefin-based elastomer, a stretchable laminate having more excellent fittability can be provided. In addition, the heat stability is further improved as compared to any other elastomer resin (e.g., a styrene-based elastomer), and hence, for example, the heat decomposition at the time of the formation of the resin into a film in the production of the stretchable laminate of the present invention can be further suppressed. In addition, the storage stability is further improved as compared to any other elastomer resin (e.g., a styrene-based elastomer), and hence the fluctuation of values for physical properties during the storage of the stretchable laminate of the present invention can be further suppressed. Further, the steps in the production of the elastomer layer can be further simplified, and hence the processing cost can be further suppressed.

Of the α-olefin-based elastomers that may be adopted as the elastomer resin in the present invention, an ethylene-based elastomer or a propylene-based elastomer is particularly preferred. When the ethylene-based elastomer or the propylene-based elastomer is adopted as the olefin-based elastomer, a stretchable laminate having extremely excellent fittability can be provided. In addition, the heat stability is still further improved as compared to any other elastomer resin (e.g. , a styrene-based elastomer), and hence, for example, the heat decomposition at the time of the formation of the resin into a film in the production of the stretchable laminate of the present invention canbe still further suppressed. Inaddition, the storage stability is still further improved as compared to any other elastomer resin (e.g., a styrene-based elastomer), and hence the fluctuation of values for physical properties during the storage of the stretchable laminate of the present invention can be still further suppressed. Further, the steps in the production of the elastomer layer can be still further simplified, and hence the processing cost can be still further suppressed.

The α-olefin-based elastomer is also available as a commercial product. Examples of such commercial product include some products in the "Tafmer" (trademark) series (e.g. , Tafmer PN-2070 and Tafmer PN-3560) manufactured by Mitsui Chemicals, Inc., and some products in the "Vistamaxx" (trademark) series (e.g., Vistamaxx 3000, Vistamaxx 6202, and Vistamaxx 7010) manufactured by Exxon Mobil Corporation.

The α-olefin-based elastomer that may be adopted as the elastomer resin in the present invention is preferably produced by using a metallocene catalyst. When the α-olefin-based elastomer produced by using a metallocene catalyst is adopted, a stretchable laminate having extremely excellent fittability can be provided. In addition, the heat stability is still further improved as compared to any other elastomer resin (e.g., a styrene-based elastomer), and hence, for example, the heat decomposition at the time of the formation of the resin into a film in the production of the stretchable laminate of the present invention can be still further suppressed. In addition, the storage stability is still further improved as compared to any other elastomer resin (e.g., a styrene-based elastomer), and hence the fluctuation of values for physical properties during the storage of the stretchable laminate of the present invention can be still further suppressed. Further, the steps in the production of the elastomer layer can be still further simplified, and hence the processing cost can be still further suppressed.

The elastomer layer may contain any appropriate other component as long as the effects of the present invention are not impaired. Examples of such other component include any other polymer, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. The number of kinds of those components may be only one, or may be two or more. The content of the other component in the elastomer layer is preferably 10 wt% or less, more preferably 7 wt% or less, still more preferably 5 wt% or less, particularly preferably 2 wt% or less, most preferably 1 wt% or less.

The thickness of the elastomer layer is preferably from 20 µm to 200 µm, more preferably from 30 µm to 160 µm, still more preferably from 30 um to 140 µm, particularly preferably from 30 um to 120 µm, most preferably from 30 µm to 100 µm. When the thickness of the elastomer layer falls within such range, a stretchable laminate having more excellent fittability can be provided.

### <<Non-woven Fabric Layer>>

The stretchable laminate of the present invention is formed of more than two layers including the elastomer layer, and includes a non-woven fabric layer as a layer except the elastomer layer. A layer adjacent to the elastomer layer is the non-woven fabric layer.

Any appropriate non-woven fabric layer may be adopted as the non-woven fabric layer as long as the effects of the present invention are not impaired. The number of kinds of non-woven fabrics constituting the non-woven fabric layer may be only one, or may be two or more.

Examples of the non-woven fabric constituting the non-woven fabric layer include a spunbonded non-woven web, a fluffy non-woven fabric (such as a non-woven fabric obtained by a thermal bonding method, a bonding joining method, or a spunlace method), a meltblown non-woven web, a spunlace non-woven web, a spunbonded meltblown spunbonded non-woven web, a spunbonded meltblown meltblown spunbonded non-woven web, an unjoined non-woven web, an electrospun non-woven web, a flashspun non-woven web (such as TYVEKTM from Du Pont), and a carded non-woven fabric.

For example, the non-woven fabric constituting the non-woven fabric layer may contain fibers of polypropylene, polyethylene, polyester, polyamide, polyurethane, an elastomer, rayon, cellulose, acrylic, a copolymer thereof, or a blend thereof, or a mixture thereof, or any other polyolefin. The non-woven fabric preferably contains fibers of polyolefin, such as polypropylene or polyethylene, out of those fibers because the effects of the present invention can be expressed to a larger extent.

The non-woven fabric constituting the non-woven fabric layer may contain fibers as a homogeneous structural body, or may contain a bicomponent structural body, such as a sheath/core structure, a side-by-side structure, a sea-island structure, and any other bicomponent structure. Detailed descriptions of the non-woven fabric may be found in, for example, "Nonwoven Fabric Primer and Reference Sampler, " E. A. Vaughn, Association of the Nonwoven Fabrics Industry, third edition (1992).

The basis amount of the non-woven fabric constituting the non-woven fabric layer is preferably 150 gsm or less, more preferably 100 gsm or less, still more preferably 50 gsm or less, particularly preferably from 10 gsm to 30 gsm.

### <<<<Production of Stretchable Laminate>>>>

Any appropriate production method may be adopted as a method of producing the stretchable laminate of the present invention to the extent that the effects of the present invention are not impaired as long as a stretchable laminate in which the elastomer layer 10, the non-woven fabric layer 20a, and the non-woven fabric layer 20b are directly laminated can be produced by the method. Such production method is a method in which the elastomer layer 10,the non-woven fabric layer 20a, and the non-woven fabric layer 20b are directly fused and bonded to each other described in detail in the foregoing, and the welding bonding is ultrasonic welding bonding.

Since the stretchable laminate of the present invention is such stretchable laminate as illustrated in each of FIG. 3 and FIG. 4, the laminate has the region C having the through-holes. The stretchable laminate of the present invention having such construction is produced by feeding an ultrasonic wave from a horn so that vibration energy is more intensively applied to sites where the through-holes are to be formed at the time of bonding in the region C.

When the stretchable laminate of the present invention is such stretchable laminate as illustrated in each of FIG. 5 and FIG. 6, the laminate has the region A free of a through-hole in one end portion thereof and the region B free of a through-hole in the other end portion thereof, and has the region C having the through-holes between the region A and the region B. The stretchable laminate of the present invention having such construction is produced by: feeding an ultrasonic wave from a horn so that vibration energy is uniformly applied to the entirety of a bonding surface at the time of bonding in the region A and the region B; and feeding an ultrasonic wave from the horn so that vibration energy is more intensively applied to sites where the through-holes are to be formed at the time of bonding in the region C.

The stretchable laminate of the present invention may be subjected to treatments referred to as pre-stretching treatment and activation treatment after the lamination. Specifically, stretching treatment is performed in a width direction of the stretchable laminate or, for example, treatment in which a fiber structure of a part of the region of the non-woven fabric layer is mechanically broken may be performed. When such treatments are performed, the stretchable laminate can be stretched by a smaller force.

### <<Application of Stretchable Laminate of the Present Invention>>

The stretchable laminate of the present invention can be used in any appropriate article in which the effects of the present invention can be effectively utilized. That is, the article of the present invention includes the stretchable laminate of the present invention. A typical example of such article is a sanitary article . Examples of such sanitary article include a diaper (in particular, an ear portion of a disposable diaper), a supporter, and a mask.

### Examples

The present invention is hereinafter specifically described by way of Examples. However, the present invention is by no means limited to these Examples. Test and evaluation methods in Examples and the like are as described below. In addition, "part (s) " means "part(s) by weight" and "%" means "wt%" unless otherwise stated.

### <Delamination Test>

Stretchable laminates obtained in Examples and Comparative Examples were each evaluated by performing a delamination test as described below. Each of the stretchable laminates was cut into a piece having a width of 30 mm and a length of 10 cm so that a cross direction (CD) vertical to a machine direction (MD) of a film served as a long side. The resultant stretchable laminate was set in a tension testing machine (manufactured by Shimadzu Corporation: AG-IS 50 kN) with a rubber plate so that a distance between chucks became 50 mm, and the laminate was stopped at a tension speed of 300 mm/min and a moving distance of 50 mm (100% extension) . After having been pulled for 30 minutes in its held state, the stretchable laminate was removed and the state of the peeling (delamination) of a non-woven fabric was observed.

A stretchable laminate in which 1 cm² or more of the non-woven fabric peeled from an elastic film was evaluated as ×, and any other stretchable laminate was evaluated as o.

### <Air Permeability Test>

Air permeability was measured with an Oken-type air permeability meter (sec/100 cc) (manufactured by Asahi Seiko Co. , Ltd. , product name: EG01-7-7MR) . A stretchable laminate having an air permeability of more than 99,999 sec/100 cc was evaluated as ×, and any other stretchable laminate was evaluated as o.

### <Method of evaluating Chemical Substance Odor>

The level of an odor generated from a stretchable laminate stored for 1 week at room temperature after its production was subjected to a sensory evaluation in accordance with the following evaluation criteria.
×: Odor easily sensed
Δ: Weak odor
o: No odor

### <Elastomer Layer Forming Conditions>

In Examples and Comparative Examples, an elastomer layer (hereinafter sometimes referred to as elastic film) was formed by extrusion molding by extruding three layers in two types (A layer/B layer/A layer) through use of a T-die molding machine . The extrusion temperatures were set under the following conditions.
A layer: 200°C
B layer: 200°C
Die temperature: 200°C

### <Lamination of Non-woven Fabric Layer>

### (Ultrasonic Welding Bonding)

A stretchable laminate was obtained by performing ultrasonic welding lamination with an ultrasonic welding facility (manufactured by Herrmann, apparatus name: MICROBOND (ULTRABOND 48:20)) at a frequency of 20 kHz (output intensity: 1,800 W) and a line velocity of 400 m/min under a state in which three layers, i.e., a non-woven fabric, an elastic film, and a non-woven fabric were laminated.

### (Bonding with Hot-melt Pressure-sensitive Adhesive)

A stretchable laminate was obtained by bonding each of both surfaces of an elastic film described in each of Examples and Comparative Examples, and a non-woven fabric having a hot-melt pressure-sensitive adhesive described in each of Comparative Examples applied thereto in a stripe manner (width of a pressure-sensitive adhesive layer: 1 mm, interval: 1 mm, application amount: 8 g/m²) to each other on a roll.

### [Example 1]

90 Parts by weight of an olefin-based resin (manufactured by Exxon Mobil Corporation, product name: Vistamaxx 6202) and 10 parts by weight of an olefin-based resin (manufactured by BOREALIS, product name: Borstar (trademark) FB2230, LLDPE) were loaded into an A layer in an extrusion machine, and a formulation of 65 parts by weight of an olefin-based resin (manufactured by Exxon Mobil Corporation, product name: Vistamaxx 6202), 30 parts by weight of an olefin-based resin (manufactured by Mitsui Chemicals, Inc., product name: Tafmer PN-3560), and 5 parts by weight of a white pigment (titanium oxide, manufactured by Du pont, product name: Ti-Pure R103) was loaded into a B layer in the extrusion machine to extrude an elastic film (1) having the construction of A layer/B layer/A layer=9 µm/42 µm/9 µm in total of 60 µm.

Next, ultrasonic welding bonding was performed so that a non-woven fabric (PP carded type, basis weight=24 gsm) was directly laminated on each of both surfaces of the resultant elastic film (1), and so that when a roll of a stretchable laminate to be obtained was cut, in each of regions A and B having lengths of 10 mm each from both ends of the cut product, the non-woven fabrics and the film were completely bonded to each other so as not to have any through-holes, and in a region C between the region A and the region B, the non-woven fabrics and the film were bonded to each other so as to have through-holes each having a hole diameter (diameter) of 1.5 mm at a pitch of 8 mm. Thus, a stretchable laminate (1) was obtained.

The results are shown in Table 1.

### [Example 2]

A stretchable laminate (2) was obtained in the same manner as in Example 1 except that an elastic film (2) having the construction of A layer/B layer/A layer=6.75 µm/31.5 µm/6.75 µm in total of 45 µm was extruded.

The results are shown in Table 1.

### [Example 3]

A stretchable laminate (3) was obtained in the same manner as in Example 1 except that a non-woven fabric (PP spunlace type, basis weight=30 gsm) was used instead of the non-woven fabric (PP carded type, basis weight=24 gsm).

The results are shown in Table 1.

### [Example 4]

A stretchable laminate (4) was obtained in the same manner as in Example 2 except that a non-woven fabric (PP spunlace type, basis weight=30 gsm) was used instead of the non-woven fabric (PP carded type, basis weight=24 gsm).

The results are shown in Table 1.

### [Example 5]

A stretchable laminate (5) was obtained in the same manner as in Example 1 except that a non-woven fabric (PP spunbonded type, basis weight=20 gsm) was used instead of the non-woven fabric (PP carded type, basis weight=24 gsm).

The results are shown in Table 1.

### [Example 6]

A stretchable laminate (6) was obtained in the same manner as in Example 2 except that a non-woven fabric (PP spunbonded type, basis weight=20 gsm) was used instead of the non-woven fabric (PP carded type, basis weight=24 gsm).

The results are shown in Table 1.

### [Example 7]

70 Parts by weight of an olefin-based resin (manufactured by Exxon Mobil Corporation, product name: Vistamaxx 3000) and 30 parts by weight of an olefin-based resin (manufacturedbyBOREALIS, product name: Borstar (trademark) FB2230, LLDPE) were loaded into an A layer in an extrusion machine, and a formulation of 25 parts by weight of an olefin-based resin (manufactured by Exxon Mobil Corporation, product name: Vistamaxx 3000), 70 parts by weight of an olefin-based resin (manufactured by Mitsui Chemicals, Inc., product name: Tafmer PN-3560), and 5 parts by weight of a white pigment (titanium oxide, manufactured by Du pont, product name: Ti-Pure R103) was loaded into a B layer in the extrusion machine to extrude an elastic film (7) having the construction of A layer/B layer/A layer=6 µm/48 µm/6 µm in total of 60 µm.

A stretchable laminate (7) was obtained in the same manner as in Example 1 except that the elastic film (7) was used instead of the elastic film (1).

The results are shown in Table 1.

### [Example 8]

100 Parts by weight of an olefin-based resin (manufactured by Exxon Mobil Corporation, product name: PP9513) was loaded into an A layer in an extrusion machine, and a formulation of 65 parts by weight of an olefin-based resin (manufactured by Exxon Mobil Corporation, product name: Vistamaxx 6202), 30 parts by weight of an olefin-based resin (manufactured by Mitsui Chemicals, Inc., product name: Tafmer PN-3560), and 5 parts by weight of a white pigment (titanium oxide, manufactured by Du pont, product name: Ti-Pure R103) was loaded into a B layer in the extrusion machine to extrude an elastic film (8) having the construction of A layer/B layer/A layer=6 µm/48 µm/6 µm in total of 60 µm.

A stretchable laminate (8) was obtained in the same manner as in Example 1 except that the elastic film (8) was used instead of the elastic film (1).

The results are shown in Table 1.

### [Example 9]

100 Parts by weight of an olefin-based resin (manufactured by Exxon Mobil Corporation, product name: PP9513) was loaded into an A layer in an extrusion machine, and a formulation of 95 parts by weight of a SIS-based resin (manufactured by Zeon Corporation, product name: Quintac 3399) and 5 parts by weight of a white pigment (titanium oxide, manufactured by Du pont, product name: Ti-Pure R103) was loaded into a B layer in the extrusion machine to extrude an elastic film (9) having the construction of A layer/B layer/A layer=6 µm/48 µm/6 µm in total of 60 µm.

A stretchable laminate (9) was obtained in the same manner as in Example 1 except that the elastic film (9) was used instead of the elastic film (1).

The results are shown in Table 1.

### [Comparative Example 1]

213 Parts by weight of aSIS-basedresin (manufactured by Kraton Polymers, Inc., product name: Kraton D1165 PT), 619 parts by weight of a tackifier (manufactured by Kolon Industries, Inc., product name: SUKOREZ SU-100 S), 84 parts by weight of liquid paraffin (manufactured by Petro yag, product name: White Oil Pharma Oyster 259), and 10 parts by weight of an antioxidant (manufactured by BASF, product name: Irganox 1010) were blended to provide a hot-melt pressure-sensitive adhesive (C1).

The hot-melt pressure-sensitive adhesive (C1) was applied to each of both surfaces of the elastic film (1) obtained in Example 1, and a non-woven fabric (PP carded type, basis weight=24 gsm) was bonded to each of both surfaces of the elastic film (1) . Thus, a stretchable laminate (C1) was obtained.

The results are shown in Table 2.

### [Comparative Example 2]

100 Parts by weight of a SIS-based resin (manufactured by Zeon Corporation, product name: Quintac 3399) was loaded into an A layer in an extrusion machine, and a formulation of 95 parts by weight of a SIS-based resin (manufactured by Zeon Corporation, product name: Quintac 33 99) and 5 parts by weight of a white pigment (titanium oxide, manufactured by Du pont, product name: Ti-Pure R103) was loaded into a B layer in the extrusion machine to extrude an elastic film (C2) having the construction of A layer/B layer/A layer=9 µm/42 µm/9 µm in total of 60 µm.

Next, the hot-melt pressure-sensitive adhesive (C1) prepared in Comparative Example 1 was applied to each of both surfaces of the resultant elastic film (C2), and a non-woven fabric (PP carded type, basis weight=24 gsm) was bonded to each of both surfaces of the elastic film (C2). Thus, a stretchable laminate (C2) was obtained.

The results are shown in Table 2.

### [Comparative Example 3]

A stretchable laminate (C3) was obtained in the same manner as in Comparative Example 1 except that a non-woven fabric (PP spunlace type, basis weight=30 gsm) was used instead of the non-woven fabric (PP carded type, basis weight=24 gsm).

The results are shown in Table 2.

**Table 1**

| | | Example | | | | | | | | |
|---|---|---|---|---|---|---|---|---|---|---|
| | | 1 | 2 | 3 | 4 | 5 | 6 | 7 | 8 | 9 |
| Film formulation | | | | | | | | | | |
| A layer resin (1) | | Vistamaxx 6202 | ← | ← | ← | ← | ← | Vistamaxx 3000 | PP9513 | PP9513 |
| A layer resin (2) | | FB2230 (LLDPE) | ← | ← | ← | ← | ← | FB2230 (LLDPE) | - | - |
| B layer resin (1) | | Vistamaxx 6202 | ← | ← | ← | ← | ← | Vistamaxx 3000 | Vistamaxx 6202 | Quintac 3399 |
| B layer resin (2) | | Tafmer PN-3560 | ← | ← | ← | ← | ← | Tafmer PN-3560 | Tafmer PN-3560 | - |
| B layer resin (3) | | TiO2 MB | ← | - | ← | ← | ← | - | ← | ← |
| A layer formulation (1)/(2) | | 90/10 | ← | ← | ← | ← | ← | 70/30 | 100/0 | 100/0 |
| B layer formulation (1)/(2)/(3) | | 65/30/5 | ← | ← | ← | ← | ← | 25/70/5 | 65/30/5 | 95/0/5 |
| A/B/A thickness | µm | 9/42/9 | 6.75/31.5/6.75 | 9/42/9 | 6.75/31.5/6.75 | 9/42/9 | 6.75/31.5/6.75 | 6/48/6 | 6/48/6 | 6/48/6 |
| Thickness of elastic film layer | µm | 60 | 45 | 60 | 45 | 60 | 45 | 60 | 60 | 60 |

| Kind of non-woven fabric | | | | | | | | | | |
|---|---|---|---|---|---|---|---|---|---|---|
| Production method | | Carded | Carded | Spunlace | Spunlace | Spunbonded | Spunbonded | Carded | Carded | Carded |
| Fiber surface resin | | PP | PP | PP | PP | PP | PP | PP | PP | PP |
| Weight | g/m2 | 24 | 24 | 30 | 30 | 20 | 20 | 24 | 24 | 24 |
| | | | | Bo | nding system | | | | | |

| Kind | | Ultrasonic | Ultrasonic bonding | Ultrasonic | Ultrasonic bonding | Ultrasonic | Ultrasonic bonding | Ultrasonic | Ultrasonic | Ultrasonic |
|---|---|---|---|---|---|---|---|---|---|---|
| Interlaminar fracture failure (delamination) | | bonding ○ | ○ | bonding ○ | ○ | bonding ○ | ○ | bonding ○ | bonding ○ | bonding ○ |
| Air permeability | | ○ | ○ | ○ | ○ | ○ | ○ | ○ | ○ | ○ |
| Chemical substance odor | | ○ | ○ | ○ | ○ | ○ | ○ | ○ | ○ | Δ |

**Table 2**

| | | Comparative Example | | |
|---|---|---|---|---|
| | | 1 | 2 | 3 |
| Film formulation | | | | |
| A layer resin (1) | | Vistamaxx 6202 | Quintac 3399 | Vistamaxx 6202 |
| A layer resin (2) | | FB2230 (LLDPE) | - | FB2230 (LLDPE) |
| B layer resin (1) | | Vistamaxx 6202 | Quintac 3399 | Vistamaxx 6202 |
| B layer resin (2) | | Tafmer PN-3560 | - | Tafmer PN-3560 |
| B layer resin (3) | | TiO2 MB | - | TiO2 MB |
| A layer formulation (1)/(2) | | 90/10 | 100/0 | 90/10 |
| B layer formulation (1)/(2)/(3) | | 65/30/5 | 95/0/5 | 65/30/5 |
| A/B/A thickness | µm | 9/42/9 | 9/42/9 | 9/42/9 |
| Thickness of elastic film layer | µm | | 60 | 60 |

| Ki nd of non-woven fabric | | | | |
|---|---|---|---|---|
| Production method | | Carded | Carded | Carded |
| Fiber surface resin | | PP | PP | PP |
| | g/m2 | 24 | 24 | 30 |

| Bonding system | | | | |
|---|---|---|---|---|
| Kind | | Styrene-based HM pressure-sensitive adhesive | Styrene-based HM pressure-sensitive adhesive | Styrene-based HM pressure-sensitive adhesive |
| Interlaminar fracture failure (delamination) | | × | ○ | × |
| Air permeability | | × | × | × |
| Chemical substance odor | | × | × | × |

The stretchable laminate of the present invention can be used in any appropriate article in which the effects of the present invention can be effectively utilized. That is, the article of the present invention includes the stretchable laminate of the present invention. A typical example of such article is a sanitary article. Examples of such sanitary article include a diaper (in particular, an ear portion of a disposable diaper), a supporter, and a mask.

### Reference Signs List

- 100: stretchable laminate
- 10: elastomer layer
- 20: non-woven fabric layer
- 20a: non-woven fabric layer
- 20b: non-woven fabric layer
- 30: through-hole

## Claims

1. A stretchable laminate (100), comprising more than two layers including an elastomer layer (10), a non-woven fabric layer (20a) arranged on one side of the elastomer layer (10), and a non-woven fabric layer (20b) arranged on the elastomer layer (10) on an opposite side to the non-woven fabric layer (20a), wherein the elastomer layer (10), the non-woven fabric layer (20a), and the non-woven fabric layer (20b) are directly laminated,
wherein the stretchable laminate (100) has a region C having through-holes (30),
wherein the elastomer layer (10), the non-woven fabric layer (20a), and the non-woven fabric layer (20b) are directly welded and bonded to each other,
wherein the welding bonding comprises ultrasonic welding bonding, and
wherein the through-holes (30) in the region C are formed by feeding an ultrasonic wave from a horn so that vibration energy is more intensively applied to sites where the through-holes are to be formed at the time of bonding in the region C.

2. The stretchable laminate (100) according to claim 1, wherein the elastomer layer (10) has a three-layer structure.

3. The stretchable laminate (100) according to claim 2, wherein the three-layer structure has, as an intermediate layer, a layer in which two or more kinds of elastomers are blended, and has, as both surface layers, layers each containing one of elastomers of the same kinds as the elastomers in the intermediate layer.

4. The stretchable laminate (100) according to claim 1, wherein the elastomer layer (10) has a thickness of from 20 µm to 200 µm, preferably from 30 um to 100 µm.

5. The stretchable laminate (100) according to claim 1, wherein the elastomer layer (10) contains an olefin-based elastomer.

6. The stretchable laminate (100) according to claim 5, wherein the olefin-based elastomer comprises an α-olefin-based elastomer.

7. The stretchable laminate (100) according to claim 1, wherein the non-woven fabric layer (20a) and the non-woven fabric layer (20b) contain fibers of polyolefin.

8. The stretchable laminate (100) according to claim 1, wherein the non-woven fabric layer (20a) and the non-woven fabric layer (20b) are formed of a non-woven fabric having a basis weight of 150 gsm or less, preferably 50 gsm or less, more preferably from 10 gsm to 30 gsm.

9. The stretchable laminate (100) according to claim 1, wherein the stretchable laminate (100) has a region A free of a through-hole (30) in one end portion thereof and a region B free of a through-hole (30) in another end portion thereof, and has the region C having the through-holes (30) between the region A and the region B.

10. An article, comprising the stretchable laminate (100) of claim 1.

## Patentansprüche

1. Dehnbares Laminat (100), umfassend mehr als zwei Schichten, beinhaltend eine Elastomerschicht (10), eine Vliesstoffschicht (20a), die auf einer Seite der Elastomerschicht (10) angeordnet ist, und eine Vliesstoffschicht (20b), die auf der Elastomerschicht (10) auf einer der Vliesstoffschicht (20a) gegenüberliegenden Seite angeordnet ist, wobei die Elastomerschicht (10), die Vliesstoffschicht (20a) und die Vliesstoffschicht (20b) direkt laminiert sind,
wobei das dehnbare Laminat (100) einen Bereich C mit Durchgangslöchern (30) aufweist,
wobei die Elastomerschicht (10), die Vliesstoffschicht (20a) und die Vliesstoffschicht (20b) direkt miteinander verschweißt und verbunden sind,
wobei das Schweißverbinden Ultraschall-Schweißverbinden umfasst, und wobei die Durchgangslöcher (30) in dem Bereich C durch Zuführen einer Ultraschallwelle von einem Horn gebildet werden, so dass Schwingungsenergie intensiver auf Stellen angewendet wird, in denen die Durchgangslöcher zum Zeitpunkt des Verbindens in dem Bereich C gebildet werden sollen.

2. Dehnbares Laminat (100) nach Anspruch 1, wobei die Elastomerschicht (10) eine Dreischicht-Struktur aufweist.

3. Dehnbares Laminat (100) nach Anspruch 2, wobei die Dreischicht-Struktur als eine Zwischenschicht eine Schicht aufweist, in der zwei oder mehr Arten von Elastomeren gemischt sind, und als beide Oberflächenschichten Schichten aufweist, die jeweils eines der Elastomere der gleichen Arten wie die Elastomere in der Zwischenschicht enthalten.

4. Dehnbares Laminat (100) nach Anspruch 1, wobei die Elastomerschicht (10) eine Dicke von 20 µm bis 200 µm, vorzugsweise von 30 µm bis 100 µm, aufweist.

5. Dehnbares Laminat (100) nach Anspruch 1, wobei die Elastomerschicht (10) ein Elastomer auf Olefinbasis enthält.

6. Dehnbares Laminat (100) nach Anspruch 5, wobei das Elastomer auf Olefinbasis ein Elastomer auf α-Olefinbasis umfasst.

7. Dehnbares Laminat (100) nach Anspruch 1, wobei die Vliesstoffschicht (20a) und die Vliesstoffschicht (20b) Fasern aus Polyolefin enthalten.

8. Dehnbares Laminat (100) nach Anspruch 1, wobei die Vliesstoffschicht (20a) und die Vliesstoffschicht (20b) aus einem Vliesstoff mit einem Flächengewicht von 150 g/m² oder weniger, bevorzugt 50 g/m² oder weniger, mehr bevorzugt von 10 g/m² bis 30 g/m², gebildet sind.

9. Dehnbares Laminat (100) nach Anspruch 1, wobei das dehnbare Laminat (100) einen Bereich A frei von einem Durchgangsloch (30) in einem Endabschnitt davon und einen Bereich B frei von einem Durchgangsloch (30) in einem anderen Endabschnitt davon aufweist und den Bereich C, der Durchgangslöcher (30) aufweist, zwischen dem Bereich A und dem Bereich B aufweist.

10. Gegenstand, umfassend das dehnbare Laminat (100) nach Anspruch 1.

## Revendications

1. Stratifié extensible (100), comprenant plus de deux couches y compris une couche d'élastomère (10), une couche de tissu non tissé (20a) disposée sur un côté de la couche d'élastomère (10), et une couche de tissu non tissé (20b) disposée sur la couche d'élastomère (10) sur un côté opposé à la couche de tissu non tissé (20a), dans lequel la couche d'élastomère (10), la couche de tissu non tissé (20a) et la couche de tissu non tissé (20b) sont directement stratifiées,
dans lequel le stratifié extensible (100) présente une zone C avec des trous traversants (30),
dans lequel la couche d'élastomère (10), la couche de tissu non tissé (20a) et la couche de tissu non tissé (20b) sont directement soudées et collées l'une à l'autre, dans lequel le collage par soudage comprend un collage par soudage aux ultrasons, et
dans lequel les trous traversants (30) dans la zone C sont formés par injection d'une onde ultrasonique d'un pavillon de sorte que l'énergie vibratoire est plus intensivement appliquée à des sites où les trous traversants doivent être formés au moment du collage dans la zone C.

2. Stratifié extensible (100) selon la revendication 1, dans lequel la couche d'élastomère (10) présente une structure tri-couche.

3. Stratifié extensible (100) selon la revendication 2, dans lequel la structure tri-couche a, en tant que couche intermédiaire, une couche dans laquelle deux sortes d'élastomère, ou plus, sont mélangées et a, en tant que deux couches de surface, des couches contenant chacune l'un des élastomères des mêmes sortes que les élastomères de la couche intermédiaire.

4. Stratifié extensible (100) selon la revendication 1, dans lequel la couche d'élastomère (10) a une épaisseur allant de 20 µm à 200 µm, de préférence allant de 30 µm à 100 µm.

5. Stratifié extensible (100) selon la revendication 1, dans lequel la couche d'élastomère (10) contient un élastomère à base d'oléfine.

6. Stratifié extensible (100) selon la revendication 5, dans lequel l'élastomère à base d'oléfine comprend un élastomère à base d'a-oléfine.

7. Stratifié extensible (100) selon la revendication 1, dans lequel la couche de tissu non tissé (20a) et la couche de tissu non tissé (20b) contiennent des fibres de polyoléfine.

8. Stratifié extensible (100) selon la revendication 1, dans lequel la couche de tissu non tissé (20a) et la couche de tissu non tissé (20b) sont constituées d'un tissu non tissé présentant un poids de base de 150 gsm ou moins, de préférence de 50 gsm ou moins, plus préférablement de 10 gsm à 30 gsm.

9. Stratifié extensible (100) selon la revendication 1, dans lequel le stratifié extensible (100) présente une zone A exempte de trou traversant (30) dans une partie terminale de celle-ci et une zone B exempte de trou traversant (30) dans une autre partie terminale de celle-ci, et a la zone C avec des trous traversants (30) entre la zone A et la zone B.

10. Article comprenant le stratifié extensible (100) selon la revendication 1.
